(19) **Europäisches Patentamt** / **European Patent Office** / **Office européen des brevets**

(11) **EP 2 152 202 B1**

(12) **EUROPEAN PATENT SPECIFICATION**

(45) Date of publication and mention
of the grant of the patent:
**29.12.2010 Bulletin 2010/52**

(51) Int Cl.:
***A61F 2/16*** (2006.01)

(21) Application number: **08769234.9**

(22) Date of filing: **29.04.2008**

(86) International application number:
**PCT/US2008/061905**

(87) International publication number:
**WO 2008/137425 (13.11.2008 Gazette 2008/46)**

(54) **IOL PERIPHERAL SURFACE DESIGNS TO REDUCE NEGATIVE DYSPHOTOPSIA**

PERIPHERE OBERFLÄCHENGESTALTUNG FÜR EINE IOL ZUR VERMINDERUNG VON NEGATIVER DYSPHOTOPSIE

CONCEPTIONS DE SURFACE PÉRIPHÉRIQUE D'UNE LIO POUVANT RÉDUIRE UNE DYSPHOTOPSIE NÉGATIVE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **30.04.2007 US 741841**
**24.05.2007 US 753251**

(43) Date of publication of application:
**17.02.2010 Bulletin 2010/07**

(73) Proprietor: **Alcon, Inc.**
**6331 Hünenberg (CH)**

(72) Inventors:
• **SIMPSON, Michael J.**
**Arlington, Texas 76012 (US)**
• **STANLEY, Dan**
**Midlothian, Texas 76065 (US)**

• **ZHANG, Xiaoxiao**
**Fort Worth, Texas 76132 (US)**
• **ELLIS, K. Scott**
**Tucson, Arizona 85730 (US)**

(74) Representative: **Hanratty, Catherine et al**
**Hanna Moore & Curley**
**13 Lower Lad Lane**
**IRL-Dublin 2 (IE)**

(56) References cited:
**WO-A-03/047466          WO-A-2006/060480**
**US-A1- 2005 060 031     US-A1- 2007 067 031**

• **RADFORD ET AL: "Comparison of pseudophakic dysphotopsia with Akreos Adapt and SN60-AT intraocular lenses" JOURNAL CATARACT AND REFRACTIVE SURGERY, SURGERY, FAIRFAX, VA, vol. 33, no. 1, 22 December 2006 (2006-12-22), pages 88-93, XP005735077 ISSN: 0886-3350**

**Description**

**BACKGROUND**

The present invention relates generally to intraocular lenses (IOLs), and particularly to IOLs that provide a patient with an image of a field of view without the perception of visual artifacts in the peripheral visual field.

The optical power of the eye is determined by the optical power of the cornea and that of the natural crystalline lens, with the lens providing about a third of the eye's total optical power. The process of aging as well as certain diseases, such as diabetes, can cause clouding of the natural lens, a condition commonly known as cataract, which can adversely affect a patient's vision.

Intraocular lenses are routinely employed to replace such a clouded natural lens. Although such IOLs can substantially restore the quality of a patient's vision, some patients with implanted IOLs report aberrant optical phenomena, such as halos, glare or dark regions in their vision. These aberrations are often referred to as "dysphotopsia." In particular, some patients report the perception of shadows, particularly in their temporal peripheral visual fields. This phenomenon is generally referred to as "negative dysphotopsia"

The document US 2005060031 discloses the features of the preamble of claim 1.

Accordingly, there is a need for enhanced IOIs, especially IOLs that can reduce dysphotopsia, in general, and the perception of shadows or negative dysphotopsia, in particular.

**SUMMARY**

The present invention generally provides intraocular lenses (IOLs) in which one or more peripheral surfaces of the optic are designed to alleviate, and preferably eliminate, the perception of shadows that some IOL patients report.

The present invention is based, in part, on the discovery that the shadows perceived by IOL patients can be caused by a double imaging effect when light enters the eye at very large visual angles. More specifically, it has been discovered that in many conventional IOLs, most of the light entering the eye is focused by both the cornea and the IOL onto the retina, but some of the peripheral light misses the IOL and it is hence focused only by the cornea. This leads to the formation of a second peripheral image. Although this image can be valuable since it extends the peripheral visual field, in some IOL users it can result in the perception of a shadow-like phenomenon that can be distracting.

To reduce the potential complications of cataract surgery, designers of modem IOLs have sought to make the optical component (the "optic") smaller (and preferably foldable) so that it can be inserted into the capsular bag with greater ease following the removal of the patient's natural crystalline lens. The reduced lens diameter, and foldable lens materials, are important factors in the success of modem IOL surgery, since they reduce the size of the corneal incision that is required. This in turn results in a reduction in corneal aberrations from the surgical incision, since often no suturing is required. The use of self-sealing incisions results in rapid rehabilitation and further reductions in induced aberrations. However, a consequence of the optic diameter choice is that the IOL optic may not always be large enough (or may be too far displaced from the iris) to receive all of the light entering the eye.

Moreover, the use of enhanced polymeric materials and other advances in IOL technology have led to a substantial reduction in capsular opacification, which has historically occurred after the implantation of an IOL in the eye, e.g., due to cell growth. Surgical techniques have also improved along with the lens designs, and biological material that used to affect light near the edge of an IOL, and in the region surrounding the IOL, no longer does so. These improvements have resulted in a better peripheral vision, as well as a better foveal vision, for the IOL users. Though a peripheral image is not seen as sharply as a central (axial) image, peripheral vision can be very valuable. For example, peripheral vision can alert IOL users to the presence of an object in their field of view, in response to which they can turn to obtain a sharper image of the object. It is interesting to note in this regard that the retina is a highly curved optical sensor, and hence can potentially provide better off-axis detection capabilities than comparable flat photosensors. In fact, though not widely appreciated, peripheral retinal sensors for visual angles greater than about 60 degrees are located in the anterior portion of the eye, and are generally oriented toward the rear of the eye. In some IOL users, however, the enhanced peripheral vision can lead to, or exacerbate, the perception of peripheral visual artifacts, e.g., in the form of shadows.

Dysphotopsia (or negative dysphotopsia) is often observed by patients in only a portion of their field of vision because the nose, cheek and brow block most high angle peripheral light rays - except those entering the eye from the temporal direction. Moreover, because the IOL is typically designed to be affixed by haptics to the interior of the capsular bag, errors in fixation or any asymmetry in the bag itself can exacerbate the problem - especially if the misalignment causes more peripheral temporal light to bypass the IOL optic.

In many embodiments of the IOLs according to the teachings of the invention, a peripheral region of the IOL's posterior surface is configured to direct at least some of the light rays incident thereon (via refraction by the anterior surface and passage through the lens body) to a reduced intensity region between a secondary peripheral image, formed by rays entering the eye that miss the IOL, and an image formed by the IOL. Such redirecting of some light into the shadow region advantageously ameliorates, and preferably prevents, the perception of peripheral visual artifacts by the IOL users.

In one aspect, an IOL is disclosed that includes an anterior surface and a posterior surface disposed about an optical axis, where the posterior surface includes a central region extending to a peripheral region. Once the IOL is implanted in a patient's eye, the anterior surface and the central region of the posterior surface cooperatively form an image of a field of view on the retina and the peripheral region of the posterior surface directs at least some light rays incident thereon (e.g., via refraction by the anterior surface) to at least one retinal location offset from the image so as to inhibit dysphotopsia.

In a related aspect, the peripheral region is adapted to receive at least some of the light rays incident on the anterior surface at angles in a range of about 50 to about 80 degrees relative to the IOL's optical axis. In some embodiments, the anterior surface exhibits a radius relative to the optical axis in a range of about 2 mm to about 4.5 mm, and the central portion of the posterior surface exhibits a respective radius in a range of about 1.5 mm to about 4 mm. Further, the peripheral region can have a width in a range of about 0.5 mm to about 1 mm. The optic is preferably formed of a biocompatible material having a suitable index of refraction, e.g., in a range of about 1.4 to about 1.6.

In another aspect, a focusing power provided by a combination of the IOL's anterior surface and the central region of the posterior surface is greater than a respective focusing power provided by a combination of the anterior surface and the peripheral region of the posterior surface. By way of example, such difference in the focusing powers can be in a range of about 25% to about 75%, and preferably in a range of about 25% to about 50%.

In another aspect, in the above IOL, at least one of the anterior surface or the central region of the posterior surface exhibits an asphericity, e.g., one characterized by a conic constant in a range of about -10 to about -100.

In another aspect, an edge surface can extend between the boundaries of the anterior and the posterior surfaces. In many embodiments, the edge surface is textured (e.g., it includes surface undulations with physical surface amplitudes in a range of about 0.5 microns to about 2 microns) so as to scatter light incident thereon in order to prevent the formation of a secondary image that could exacerbate dysphotopsia. Although in this embodiment the edge surface is substantially flat, in other embodiments, it is preferably highly convex to further lower the risk of positive dysphotopsia due to internal reflection of rays incident thereon.

In yet another aspect, a diffractive structure disposed on a portion of the anterior surface or the central region of the posterior surface provides the IOL with multiple foci, e.g., a near focus and a far focus.

In another aspect, an IOL is disclosed that includes an anterior optical surface and a posterior optical surface disposed about an optical axis, where those surfaces cooperatively provide a principal focusing power for generating an image of a field of view on the retina of a patient's eye in which the IOL is implanted. An annular peripheral surface surrounds the posterior surface. The annular surface is adapted to direct, in combination with the anterior surface, some light rays incident on the anterior surface to the retina, with a secondary focusing power less than the principal power, so as to ameliorate dysphotopsia. In some cases, the secondary focusing power differs from the primary focusing power by a factor in a range of about 25% to about 75% percent, and preferably in a range of about 25% to about 50%.

While in some embodiments the posterior surface and the annular peripheral surface form a contiguous optical surface, in other embodiments, they comprise separate surfaces that are connected together. Further, while in some embodiments the anterior and posterior surface have convex shapes, in other embodiments, they have other shapes, such as concave or flat.

In yet another aspect, an IOL is disclosed that includes an anterior optical surface and a posterior optical surface, which are disposed about an optical axis. The IOL further includes an annular focusing surface that at least partially surrounds the posterior surface, where the annular focusing surface is adapted to inhibit dysphotopsia once the IOL is implanted in a subject's eye.

In a related aspect, in the above IOL, the annular focusing surface can provide any of a refractive and/or diffractive focusing power. For example, the annular focusing surface can include a diffractive structure for directing light to the patient's retina so as to ameliorate, and preferably prevent, dysphotopsia.

In another aspect, the invention provides an IOL having an anterior surface and a posterior surface. The IOL can further include one or more focusing elements that at least partially surround the posterior surface for directing some of the light incident on the IOL to the retina so as to inhibit dysphotopsia. By way of example, the focusing elements can comprise a plurality of lenslets.

In other aspect, a method of correcting vision is disclosed that includes providing an intraocular lens (IOL) for implantation in a patient's eye, where the IOL comprises an anterior optical surface and a posterior optical surface disposed about an optical axis, and the posterior surface includes an annular focusing region that is adapted to inhibit dysphotopsia. The IOL can be implanted in the patient's eye, e.g., to replace a clouded natural lens.

Further understanding of the invention can be obtained by reference to the following detailed description in conjunction with the associated drawings, which are described briefly below.

**BRIEF DESCRIPTION OF DRAWINGS**

FIGURE 1A is a schematic side view of an IOL in accordance with one embodiment of the invention.

FIGURE 1B is a schematic perspective view of the IOL of FIGURE 1A.

FIGURE 2 schematically depicts that some light rays incident on the anterior surface of the IOL of FIGURES 1A and 1B are refracted by that surface so as to reach the peripheral region of the IOL's posterior surface.

FIGURE 3 is another schematic side view of the IOL of FIGURES 1A and 1B in which the radius of the anterior surface and that of the central region of the posterior surface as well as the width of the annular peripheral region of the posterior surface are labeled.

FIGURE 4 is a schematic side view of an IOL according to one embodiment of the invention, which includes a textured edge.

FIGURE 5 schematically depicts the focusing function of the peripheral region of the posterior surface of an IOL according to the invention in ameliorating, and preferably preventing, dysphotopsia.

FIGURE 6A is a calculated point spread function (PSF) corresponding to a hypothetical conventional IOL.

FIGURE 6B is a calculated point spread function (PSF) corresponding to a hypothetical IOL according to one embodiment of the invention

FIGURE 7 is a theoretical curve depicting irradiance on the retina as a function of visual angle for a conventional IOL and two IOLs in accordance to two embodiments of the invention.

FIGURE 8 schematically depicts a cross-sectional slice of the posterior surface of the IOL of FIGURE 1A.

FIGURE 9 schematically depicts scattering of light incident on the textured edge surface of an IOL according to one embodiment of the invention.

FIGURE 10A is a schematic cross-sectional view of an IOL in accordance with another embodiment of the invention having an anterior surface, a posterior surface, and an annular diffractive peripheral region that surrounds the posterior surface.

FIGURE 10B is a schematic top view of the posterior surface and the annular diffractive region of the IOL of FIGURE 10A.

FIGURE 10C is a schematic side view of an IOL according to another embodiment of the invention having a Fresnel lens on a peripheral region of its posterior surface.

FIGURE 11A is a schematic side view of an IOL according to another embodiment of the invention.

FIGURE 11B schematically depicts the IOL of FIGURE 11A implanted in a patient's eye, further illustrating that the IOL inhibits dysphotopsia.

FIGURE 11C is a partial schematic side view of an IOL according to another embodiment of the invention similar to the IOL depicted in FIGURE 11A

FIGURE 12 is a schematic side view of a multifocal IOL according to another embodiment of the invention.

**DETAILED DESCRIPTION**

The present invention generally provides intraocular lenses that include peripheral light-directing surfaces and/or optical elements that direct at least a portion of incident light to one or more retinal locations offset from a main image formed by the TOL so as to inhibit (ameliorate and preferably prevent) peripheral visual artifacts in the IOL user's visual field. The term "intraocular lens" and its abbreviation "IOL" are used herein interchangeably to describe lenses that are implanted into the interior of the eye to either replace the eye's natural lens or to otherwise augment vision regardless of whether or not the natural lens is removed. Phakic lenses, for example, are examples of lenses that may be implanted into the eye without removal of the natural lens.

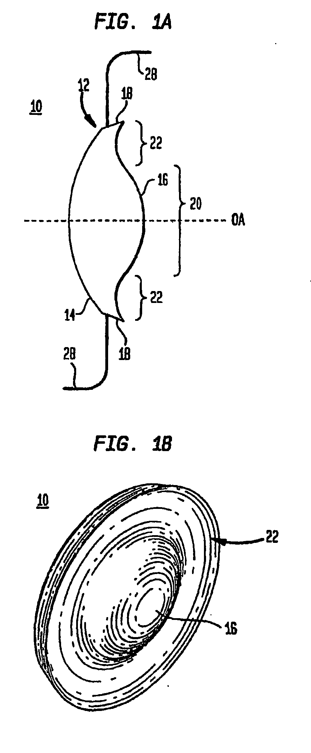

By way of example, with reference to FIGURES 1A and 1B, an intraocular lens (IOL) 10 in accordance with one embodiment of the invention includes an optic 12 disposed about an optical axis OA, which is formed of an anterior surface 14, a posterior surface 16 and an edge surface 18 that extends between the anterior and the posterior surfaces. The posterior surface 16 includes a central region 20 that extends to an annular peripheral region 22.

The anterior surface 14 and the central region 20 of the posterior surface 16 have substantially convex shapes - though other shapes are possible in other embodiments - and cooperatively provide a desired focusing power, e.g., one in a range of about -20 D to about 40 D, and preferably in a range of about -15 D to about +10 D. As discussed further below, once the IOL is implanted in a patient's eye, the optical power provided by the combination of the anterior surface and the central region of the posterior surface facilitates generation of an image of a field of view on the patient's retina.

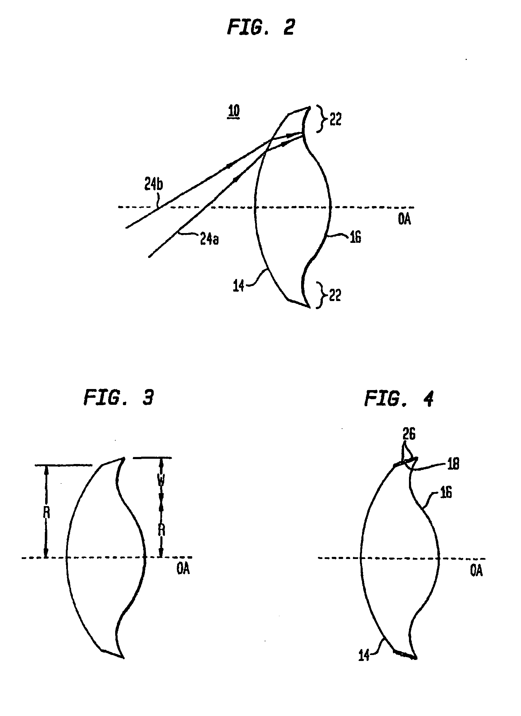

In this embodiment, the peripheral region 22 of the posterior surface 16 has, however, a substantially concave shape, and is adapted to receive peripheral light rays incident on the anterior surface at large angles relative to the optical axis OA, e.g., rays incident on the anterior surface at angles greater than about 50 degrees (e.g., in a range of about 50 degree to about 80 degrees) relative to the optical axis OA. More specifically, as shown schematically in FIGURE 2, such rays (e.g., rays 24a and 24b) are refracted by the anterior surface 14 and pass through the lens body to be incident on the peripheral region. As discussed further below, the peripheral focusing region 22 directs these light rays to one or more locations on the retina that are offset from the image formed by the anterior surface and the central region of the posterior surface so as to inhibit perception of peripheral visual artifacts (e.g., dark shadows) by the patient. To this end, in many embodiments, the refractive power provided by the combination of the anterior surface and the peripheral region of the posterior surface (herein also referred to as the IOL's secondary power) is less than the IOL's primary refractive power (that is, the refractive power provided by the anterior surface and central region of the posterior surface). By way of example, the IOL's secondary power can differ from its primary power by a factor in a range of about 25% to about 75% percent, and more preferably in a range of about 25% to about 50%. In this embodiment, the IOL's secondary power is about half of its primary power.

As shown schematically in FIGURE 3, in many embodiments, the anterior surface 14 can have a radius R relative to the optical axis OA in a range of about 2 mm to about 4.5 mm, while the central region 20 of the posterior surface 16 can have a respective radius $R^1$ in a range of about 1.5 mm to about 4 mm. The annular peripheral region 20 of the posterior surface 16 can, in turn, have a width w in a range of about 0.5 mm to about 1 mm. Further, the refractive index of the material from which the IOL is formed can be in a range of about 1.4 to about 1.6.

With reference to FIGURE 4, in some embodiments, the edge surface 18 spanning between the boundaries of the anterior surface 14 and the posterior surface 16 is textured so as to cause scattering of light incident thereon. For example, the edge surface 18 can include a plurality of surface undulations 26 with physical surface amplitudes that are of the order of wavelengths of visible light (e.g., the amplitudes of the surface undulations can be in a range of about 0.5 microns to about 2 microns).

The optic 12 is preferably formed of a biocompatible material, such as soft acrylic, silicone, hydrogel, or other biocompatible polymeric materials having a requisite index of refraction for a particular application. For example, in some embodiments, the optic can be formed of a cross-linked copolymer of 2-phenylethyl acrylate and 2-phenylethyl methacrylate, which is commonly known as Acrysof®.

Referring again to FIGURE 1A, the IOL 10 can also include a plurality of fixation members (haptics) 28 that facilitate its placement in the eye. Similar to the optic 10, the haptics 28 can also be formed of a suitable biocompatible material, such as polymethylmethacrylate. While in some embodiments the haptics can be formed integrally with the optic, in other embodiments (multipiece IOLs) the haptics are formed separately and are attached to the optic in a manner known in the art. In the latter case, the material from which the haptics are formed can be the same or different from the material forming the optic. It should be appreciated that various haptic designs for maintaining lens stability and centration are known in the art, including, for example, C-loops, J-loops, and plate-shaped haptic designs. The present invention is readily employed with any of these haptic designs.

Further, in this embodiment, the optic 10 is foldable so as to facilitate its insertion into a patient's eye, e.g., to replace a clouded natural lens.

In use, the IOL can be implanted in a patient's eye, during cataract surgery, to replace a clouded natural lens. During cataract surgery, an incision can be made in the cornea, e.g., via a diamond blade, to allow other instruments to enter the eye. Subsequently, the anterior lens capsule can be accessed via that incision to be cut in a circular fashion and removed from the eye. A probe can then be inserted through the corneal incision to break up the natural lens via ultrasound, and the lens fragments can be aspirated. An injector can be employed to place the IOL, while in a folded state, in the original lens capsule. Upon insertion, the IOL can unfold and its haptics can anchor it within the capsular bag.

In some cases, the IOL is implanted into the eye by utilizing an injector system rather than employing forceps insertion. For example, an injection handpiece having a nozzle adapted for insertion through a small incision into the eye can be used. The IOL can be pushed through the nozzle bore to be delivered to the capsular bag in a folded, twisted, or otherwise compressed state. The use of such an injector system can be advantageous as it allows implanting the IOL through a small incision into the eye, and further minimizes the handling of the IOL by the medical professional. By way of example, U.S. Patent No. 7,156,854 entitled "Lens Delivery System," discloses an IOL injector system. The IOLs according to various embodiments of the invention, such as the IOL 10, are preferably designed to inhibit dysphotopsia while ensuring that their shapes and sizes allow them to be inserted into the eye via the injector systems through small incisions.

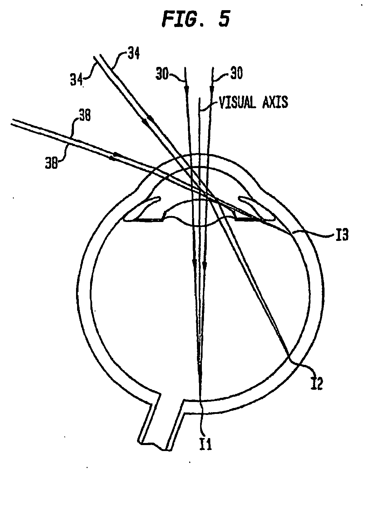

Once implanted in a patient's eye, the IOL 10 can form an image of a field of view. By way of example, with reference to FIGURE 5, a plurality of light rays, such as exemplary rays 30, emanating from a field of view can be focused by the combined optical power of the anterior surface of the IOL and that of the central region of the IOL's posterior surface to form an image 11 (herein also referred to as primary image) on the retina. In the exemplary IOL 10, the central region 20 of the posterior surface 16 has a smaller radial extension than the anterior surface so as to accommodate the incorporation of the peripheral region 22 in the IOL. The smaller size of the posterior surface's central region, however, does not lead to a substantial degradation, if any, of on-axis optical image quality. In particular, the cornea provides some focusing of the light before it reaches the IOL's anterior surface, and the anterior surface focuses the light further before it reaches the IOL's posterior surface. As a result, a substantially on-axis light beam that is incident on the cornea with a given diameter (e.g., 6 mm) has a reduced diameter at the posterior surface. As such, the peripheral region does not interfere with the focusing of such a light beam, and hence an image of a field of view with good optical quality can be obtained.

With continued reference to FIGURE 5 as well as FIGURE 1A, the peripheral region 22 of the IOL's posterior surface, in turn, receives light rays incident on the IOL's anterior surface at relatively large angles with respect to the IOL's optical axis OA (such as exemplary rays 34) and directs those rays to location(s) on the retina (such as retinal location 12) that are offset from the image I1 so as to inhibit dysphotopsia. The focusing function of the peripheral region in ameliorating, and preferably preventing dysphotopsia, can be better understood by considering that some peripheral light rays, such as rays 38, that enter the eye at large visual angles (e.g., at angles greater that about 50 degrees relative to the eye's visual axis, e.g., in a range of about 50 degrees to about 80 degrees) may miss the IOL. As such, those rays are refracted only by the cornea and hence can be incident on a peripheral portion of the retina to form a secondary image (such as schematically-depicted image I3). This double imaging effect can give rise to the perception of a shadow-like phenomenon by some patients. To alleviate this effect, the peripheral region of the posterior surface directs some of the rays incident on the IOL to the shadow region between the two images. More specifically, as discussed above, some light rays that are peripherally incident on the anterior surface of the IOL are refracted by that surface to reach, via passage through the lens body, the peripheral region, which in turn refracts those rays further so as to direct them to the retinal reduced intensity (shadow) region.

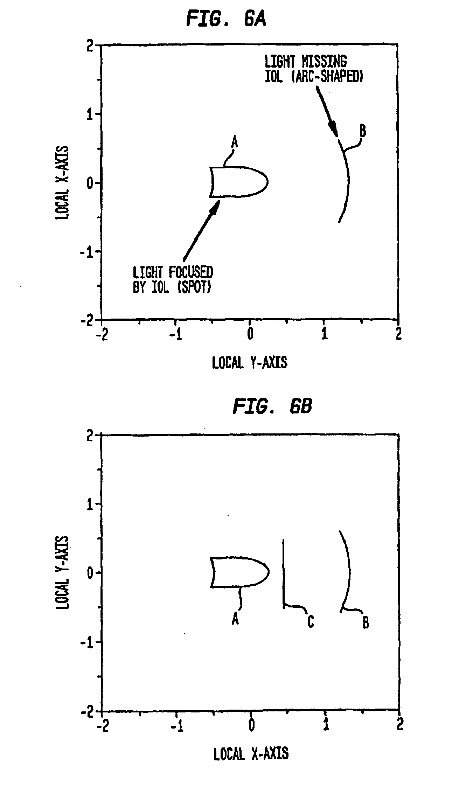

By way of further illustration, FIGURE 6A shows a calculated point spread function (PSF) on the peripheral retina of a pseudophakic eye in which a conventional IOL is implanted. The PSF corresponds to an image formed by light from a distant point source at a large visual angle. The exemplary PSF includes two components: a central component A corresponding to light focused by the combined focusing power of the cornea and the IOL (e.g., a total power of about 60 D), and a peripheral component B corresponding to light that misses the IOL and is focused only by the focusing power of the cornea (e.g., a power of about 44 D). In this example, only one peripheral component corresponding to light entering the eye from the temporal side is shown, as the nose, eyebrows, and cheeks generally prevent the formation of such shadows by light traveling in other directions. The presence of these two components creates an intermediate shadow region, which can be perceived as a shadow when a large object is seen in peripheral vision. The shadow is peripheral, e.g., in this case at a visual angle of about 70 degrees and it is typically perceived in the region of the equator of the eye globe, where the retina is relatively perpendicular to the incoming light. Shadows are generally perceived for large objects (e.g., typically with smaller pupils under bright light conditions), rather than point sources. In other words, the shadow is created by addition of the PSFs corresponding to different points of the object. Further, the long, thin crescent shape of the PSF tends to enhance the visibility of a vertical shadow, which some IOL users describe as crescent-shaped.

In contrast, FIGURE 6B shows a calculated PSF on the retina of a pseudophakic eye in which an IOL according to an embodiment of the invention, such as the above IOL 10, is implanted. Similar to the PSF shown in FIGURE 6A for a conventional IOL, this PSF also includes a central component A as well as a peripheral component B. However, this PSF further includes an intermediate component C, which is located in the gap between the central and the peripheral components. The intermediate PSF component is generated by the combined focusing function of the IOL's anterior surface and the peripheral region of its posterior surface. While this intermediate PSF component has no substantial effect on axial imaging, it alleviates, and preferably eliminates, the perception of a shadow.

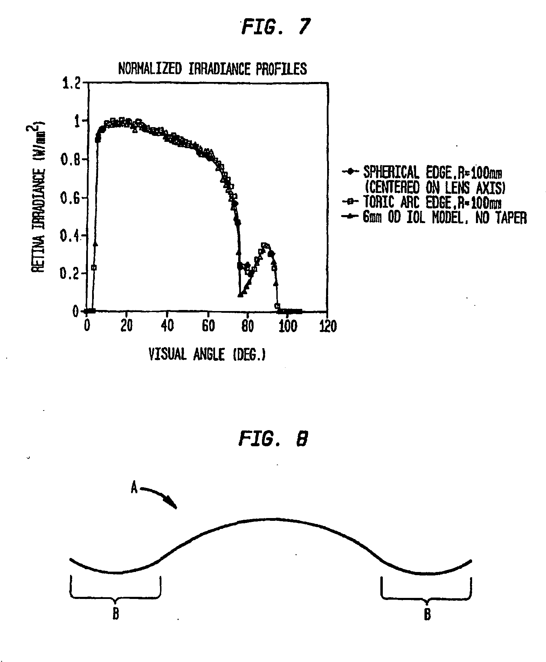

By way of further illustration of the focusing function of the peripheral region of an IOL of the invention in alleviating the perception of dark shadows, FIGURE 7 provides a theoretical comparison of retinal irradiance versus visual angle between a hypothetical conventional IOL and two exemplary hypothetical IOLs according to two embodiments of the invention. The curve corresponding to the conventional IOL (shown by solid triangles) shows a dip at a visual angle of about 75 degrees, which can lead to perception of a shadow. In contrast, the curves corresponding to IOLs of the invention (the curve shown by solid spheres corresponds to an IOL having a substantially spherical peripheral annular region and the one shown by open squares corresponds to an IOL having a toric peripheral annular region) show the depth of the shadow (i.e., the depth of the dip at a visual angle of about 75 degrees) is reduced by about 50%. This reduction can alleviate, and in many cases eliminate, the perception of a shadow by the patient. In fact, even modest reductions in conditions that create dark shadows are expected to eliminate their perception.

The annular peripheral region of the IOL 10 can have a variety of different surface profiles. For example, FIGURE 8 schematically shows a cross-sectional slice A of the IOL's posterior surface in a plane that contains the optical axis OA. In some embodiments, a curve B characterizing the cross-sectional profile of the peripheral region can be in the form of a semi-circle. Alternatively, in some cases, the curve B can exhibit an increasing deviation from circularity as a function of increasing distance from the optical axis OA. In other embodiments, the curve A can be substantially parabolic, or take any other suitable shape.

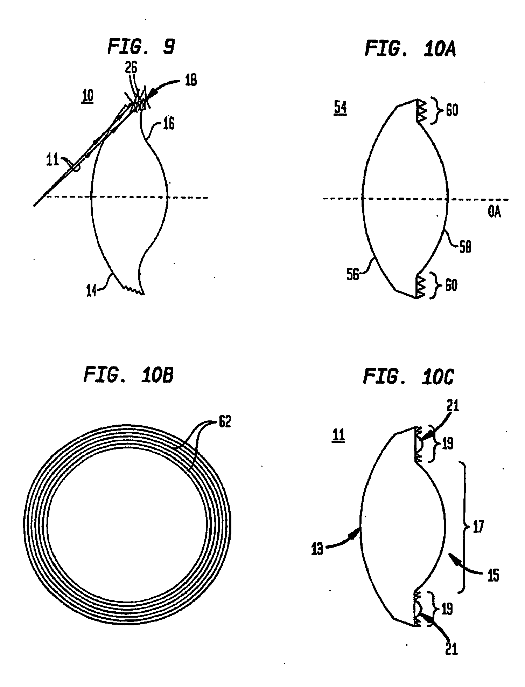

With reference to FIGURES 4 and 9, as noted above, in some embodiments the edge surface 18 is textured, e.g., it includes a plurality of surface undulations 26. The textured surface can cause scattering of light rays, such as rays 11, which are refracted by the anterior surface 14 to be incident thereon. Such scattering of the light by the textured surface ameliorates, and preferably eliminates, the possibility that some of the light incident on the edge surface would undergo total internal reflection and be subsequently refracted by the posterior surface 16 to form a secondary image on the retina. Such a secondary image could cause the perception of a dark shadow by the patient - this phenomenon is typically referred to as positive dysphotopsia. Hence, the texturing of the edge surface can preferably prevent such positive dysphotopsia. In addition, in some implementations, the edge surface is highly convex.

Some embodiments of the invention provide an IOL that includes a diffractive posterior peripheral region that sends some of the light incident on the IOL into the shadow region so as to ameliorate, and preferably prevent, dysphotopsia. By way of example, FIGURES 10A and 10B schematically depict such an IOL 54 that includes an anterior surface 56 and a posterior surface 58 that cooperatively provide a desired optical power, e.g., in a range of about -15 D to about 40 D, which is herein referred to as the IOL's primary power. A diffractive structure 60 forms an annular peripheral region that surrounds the posterior surface 58. Further an edge surface 61, which is preferably textured, connects the anterior surface to the outer boundary of the peripheral region. Though not shown, the IOL 54 can also include a plurality of fixation members (haptics) that facilitate its placement in the eye.

In this embodiment, the diffractive structure 60 is formed of a plurality of diffractive zones 62, each of which is separated from an adjacent zone by a step. In this embodiment, the step heights are uniform - although non-uniform step heights are also possible in other embodiments - and can be represented by the following relationship:

$$\text{Step height} = \frac{\lambda}{a(n_2 - n_1)} \qquad \text{Equation (1)}$$

wherein

$\lambda$ denotes a design wavelength (e.g., 550 nm),
$a$ denotes a parameter that can be adjusted to control diffraction efficiency associated with various orders, e.g., $a$ can be selected to be 1;
$n_2$ denotes the index of refraction of the optic,
$n_1$ denotes the refractive index of a medium in which the lens is placed.

Although in this embodiment, the diffractive peripheral region has a substantially flat base profile; in other embodiments the base profile can be curved. In use, the diffractive structure 60 receives some of the peripheral light rays incident on the anterior surface, e.g., rays that are incident on the anterior surface at angles in a range of about 50 to about 80 degrees relative to the optical axis OA. The diffractive structure directs at least some of those rays to a region of the retina that is offset relative to an image formed by the IOL's primary power (e.g., to a shadow region between a secondary image formed by peripheral rays entering the that miss the IOL and an image formed by the IOL) so as to inhibit dysphotopsia. To this end, in some cases, the diffractive structure, together with the anterior surface, provides an optical power that is less than the IOL's primary power by a factor in a range of about 25% to about 75%, and preferably in a range of about 25% to about 50%.

With reference to FIGURE 10C, an IOL 11 according to another embodiment includes an anterior surface 13 and a posterior surface 15 that extends from a central portion 17 to a peripheral portion 19. A Fresnel lens 21 is disposed on the peripheral portion of the posterior surface. The Fresnel lens is adapted to direct light incident thereon to the retinal shadow region between an image formed by the anterior surface and the central portion of the posterior surface and a second peripheral image that can be formed by peripheral rays entering the eye that miss the IOL. In some implementations, the optical power provided by the combination of the anterior surface and the Fresnel lens is less than the optical power provided by the anterior surface and the central portion of the posterior surface, e.g., by a factor in a range of about 25% to about 75%.

In some cases, the image quality of the primary image (the image formed by the IOL's anterior surface and the central region of its posterior surface) can affect the perception of shadows. Hence, in some embodiments, the anterior surface and/or the central portion of the posterior surface can exhibit a degree of asphericity and/or toricity. Additional teachings regarding the use of aspheric and/or toric surfaces in IOLs, such as various embodiments discussed herein, can be found in U.S. Patent Application No. 11/000,728 entitled "Contrast-Enhancing Aspheric Intraocular lens," filed on December 1, 2004 and published as Publication No. 2006/0116763.

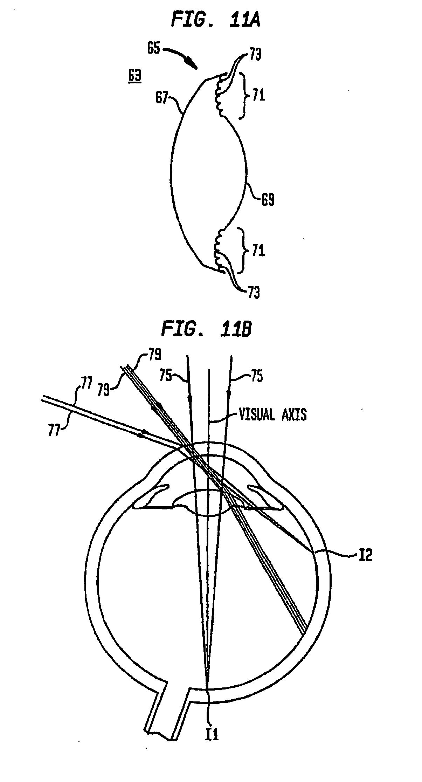

In some embodiments, the peripheral region of the IOL's posterior surface includes a plurality of lenslets, e.g., in the form of focusing surfaces positioned adjacent to one another, each of which can direct light incident thereon onto a portion of the shadow region By way of example, FIGURE 11A schematically depicts an IOL 63 according to such an embodiment that includes an optic 65 having an anterior optical surface 67 and a posterior surface optical surface 69. An annular region 71 surrounding the posterior surface includes a plurality of lenslets 73, in the form of curved surfaces. The radial dimensions of the anterior surface, the posterior surface and the width of the annular region can be similar to those provided above in connection with the previous embodiments. As shown schematically in FIGURE 11B, once implanted in the eye, the combination of the anterior and posterior surfaces can form an image I1 on the eye's retina by focusing a plurality of light rays (such as exemplary rays 75) emanating from a field of view. Some peripheral light rays (such as exemplary rays 77) may miss the IOL to form a secondary image I2. The lenslets 73, however, can redirect light rays incident thereon (such as exemplary rays 79) via refraction by the anterior surface to retinal locations between the images I1 and I2 so as to inhibit the perception of a shadow by the subject in her peripheral visual field. To this end, the combined optical power of the IOL's anterior surface and each of the lenslets is preferably less than the combined optical power of anterior and the posterior surface, e.g., by a factor in a range of about 25% to about 75%.

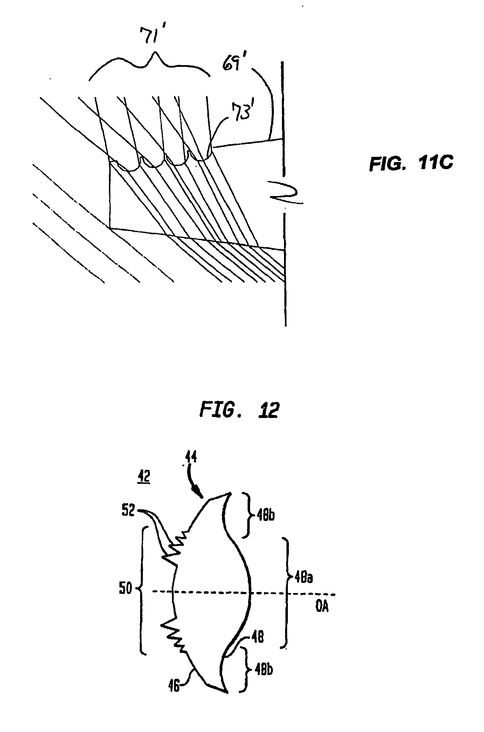

Alternatively, as best seen in FIGURE 11C, a plurality of optical grooves 73' designed as negative power or divergent refractive optics may be formed in annular region 71' on posterior optical surface 69'. The divergent optics create a diluted light distribution on the retina for the light passing through them, which softens the contrast of the light intensity distribution and consequently reduces the occurrence of negative dysphotopsia. Optics grooves 73' can be added to the normal single base curvature of the posterior optical surface 69' as shown in FIGURE 11C.

In some embodiments, a diffractive structure is disposed on the IOL's anterior surface or the central region of its posterior surface so as to provide a multifocal IOL, e.g., one having a far-focus as well as a near-focus optical power. For example, FIGURE 12 schematically depicts an IOL 42 in accordance with such embodiment that includes an optic 44 having an anterior surface 46 and posterior surface 48, which is characterized by a central region 48a and a peripheral region 48b. The peripheral region is adapted to ameliorate, and preferably prevent, dysphotopsia in a manner discussed above. A diffractive structure 50 is disposed on the anterior surface 44. The diffractive structure 50 includes a plurality of diffractive zones 52 that are separated from one another by a plurality of steps that exhibit a decreasing height as a function of increasing distance from the optical axis OA - though in other embodiments the step heights can be uniform. In other words, in this embodiment, the step heights at the boundaries of the diffractive zones arc "apodized" so as to modify the fraction of optical energy diffracted into the near and far foci as a function of aperture size (e.g., as the aperture size increases, more of the light energy is diffracted into the far focus). By way of example, the step height at each zone boundary can be defined in accordance with the following relation:

$$\text{Step height} = \frac{\lambda}{a(n_2 - n_1)} f_{apodize} \qquad \text{Equation (3)}$$

wherein

$\lambda$ denotes a design wavelength (e.g., 550 nm),
$a$ denotes a parameter that can be adjusted to control diffraction efficiency associated with various orders, e.g., $a$ can be selected to be 1.9;
$n_2$ denotes the index of refraction of the optic,
$n_1$ denotes the refractive index of a medium in which the lens is placed, and

$f_{apodize}$ represents a scaling function whose value decreases as a function of increasing radial distance from the intersection of the optical axis with the anterior surface of the lens. By way of example, the scaling function $f_{apodize}$ can be defined by the following relation:

$$f_{apodize} = 1 - \left(\frac{r_i}{r_{out}}\right)^3 \qquad \text{Equation (4).}$$

wherein

$r_i$ denotes the radial distance of the i$^{th}$ zone,
$r_{out}$ denotes the outer radius of the last bifocal diffractive zone. Other apodization scaling functions can also be employed, such as those disclosed in a co-pending patent application entitled "Apodized Aspheric Diffractive Lenses," filed December 1, 2004 having a serial number 11/000770, and published as WO 2006/060480.

In this exemplary embodiment, the diffractive zones are in the form of annular regions, where the radial location of a zone boundary ($r_i$) is defined in accordance with the following relation;

$$r_i^2 = (2i + 1)\lambda f \qquad \text{Equation (5)}$$

wherein

$i$ denotes the zone number (i = 0 denotes the central zone),
$r_i$ denotes the radial location of the i$^{th}$ zone,
$\lambda$ denotes the design wavelength, and
$f$ denotes an add power.

In many embodiments, the IOL 42 provides a far-focus optical power in a range of about -15 D to about 40 D and a near-focus optical power in a range of about 1 to about 4 D, and preferably in a range of about 2 to about 3 D. Further teachings regarding apodized diffractive lenses can be found in U.S. Patent No. 5,699,142 entitled "Diffractive Multifocal Ophthalmic Lens".

It should be understood that various changes can be made to the above embodiments without departing from the scope of the invention.

## Claims

1. An intraocular lens (IOL) (10, 1 1, 42, 54, 63), comprising:

   an anterior optical surface (14, 13, 46, 58, 67) and a posterior optical surface (16, 15, 48, 58, 69) disposed about an optical axis, said posterior surface having a central region extending to a peripheral region (22, 19, 48b, 60, 71, 71'),
   wherein the anterior surface (14, 13, 46, 58, 67) and said central region are adapted to cooperatively form an image of a field of view on the retina and said peripheral region (22, 19, 48b, 60, 71, 71') is adapted to direct some light rays incident on the anterior surface to at least one location offset from said image so as to inhibit perception of visual artifacts in a peripheral visual field, **characterised in that** said location is a retinal location.

2. The IOL of claim 1, wherein a primary focusing power provided by a combination of said anterior surface (14, 13, 46, 58, 67) and said central region of the posterior surface (16, 15 , 48, 58, 69) is greater than a respective secondary focusing power provided by a combination of said anterior surface and said peripheral region of the posterior surface.

3. The IOL of claim 1, wherein said anterior surface exhibits a radius (14, 13, 46, 58, 67) relative to said optical axis in a range of about 2 mm to about 4.5 mm.

4. The IOL of claim 3, wherein said central region of the posterior surface (16, 15, 48, 58, 69) exhibits a radius relative to said optical axis in a range of about 1.5 mm to about 4 mm.

5. The IOL of claim 4, wherein said peripheral region (22, 19, 48b, 60, 71) has a width in a range of about 0.5 mm to about 1 mm.

6. The IOL of claim 4, wherein at least one of said anterior surface or said central region of the posterior surface exhibits an asphericity **characterized by** a conic constant in a range of about -10 to about -100.

7. The IOL of claim 1, further comprising an edge surface (18, 61) extending between boundaries of said anterior and posterior surfaces.

8. The IOL of claim 1, wherein said edge surface (18, 61) is textured so as to diffuse light incident thereon.

9. The IOL of claim 8, wherein said textured edge surface (18, 61) comprises a plurality of surface undulations (26) having physical surface amplitudes in a range of about 0.5 microns to about 2 microns.

10. The IOL of claim 1, further comprising a Fresnel lens (21) disposed on said peripheral region of the posterior surface.

11. The IOL of claim 1, further comprising a diffractive structure (60) disposed on said peripheral region of the posterior surface.

12. An intraocular lens (IOL), as claimed in claims 1 or 11
wherein said anterior surface (14, 13, 46, 58, 67) and said central region cooperatively provide multiple focusing powers and said peripheral region (22, 19, 48b, 60, 71) of the posterior surface (16, 15, 48, 58, 69) is adapted to direct at least some light rays incident thereon to a retinal location between an image formed by the anterior surface and the central region and a second peripheral image formed by light rays entering the IOL that miss the IOL so as to inhibit the perception of peripheral visual artifacts.

13. An intraocular lens (IOL), as claimed in claim 1 wherein
the peripheral region (22, 19, 48b, 60, 71) comprises an annular peripheral surface at least partially surrounding said posterior surface (16, 15 , 48, 58, 69), and said anterior surface and said posterior surface cooperatively providing a principal focusing power for generating an image of a field of view on the retina of a patient's eye in which the IOL is implanted, and wherein said annular peripheral surface is adapted to direct, in combination with said anterior surface, some light rays incident on the anterior surface to the retina with a secondary focusing power less than said principal power so as to inhibit perception of visual artifacts in a peripheral visual field.

14. The IOL of claim 1 or 13, wherein said peripheral region is adapted to receive at least some of the light rays that are incident on the anterior surface at angles in a range of about 50 to about 80 degrees relative to the optical axis.

15. The IOL of claim 13, wherein said anterior surface and said posterior surface have substantially convex shapes.

16. The IOL of claim 15, wherein said annular peripheral surface has a substantially concave shape.

17. The IOL of claims 2 or 13, wherein said secondary focusing power differs from said primary focusing power by a factor in a range of about 25% to about 75%.

18. The IOL of claim 13, wherein said secondary focusing power comprises a diffractive focusing power.

19. The IOL of claim 13, wherein said annular peripheral surface and said posterior surface form a contiguous optical surface.

20. An intraocular lens (IOL), as claimed in claim 1
wherein the peripheral region (22, 19, 48b, 60, 71, 71') comprises an annular focusing surface surrounding said posterior surface, and wherein said annular focusing surface is adapted to inhibit perception of peripheral visual artifacts once the IOL is implanted in a patient's eye.

21. The IOL of claim 20, wherein said annular focusing surface directs light incident thereon to one or more retinal locations offset from an image of a field of view formed cooperatively by said anterior and posterior surfaces.

**22.** The IOL of claim 20, wherein said annular focusing surface provides a refractive focusing power or a diffractive focusing power.

**23.** The IOL of claim 20, wherein said annular focusing surface comprises a diffractive structure (60) for providing said diffractive focusing power, or, a Fresnel lens (21).

**24.** An intraocular lens (IOL) (63), as claimed in claim 1 wherein
the peripheral region (71, 71') comprises one or more focusing elements at least partially surrounding the posterior surface for directing light to the retina so as to inhibit perception of visual artifacts in a peripheral visual field.

**25.** The IOL of claim 24, wherein said focusing elements comprise lenslets (73) or optical grooves (73').


**Patentansprüche**

**1.** Intraokulare Linse (IOL) (10, 11, 42, 54, 63), umfassend:

eine anteriore optische Oberfläche (14, 13, 46, 58, 67) und eine posteriore optische Oberfläche (16, 15, 48, 58, 69), die um eine optische Achse herum angeordnet sind, wobei die posteriore Oberfläche einen mittleren Bereich aufweist, der sich bis zu einem peripheren Bereich (22, 19, 48b, 60, 71, 71') erstreckt, wobei die anteriore Oberfläche (14, 13, 46, 58, 67) und der mittlere Bereich dazu geeignet sind, um zusammenwirkend ein Bild eines Sehfeldes auf der Netzhaut zu bilden, und der periphere Bereich (22, 19, 48b, 60, 71, 71') dazu geeignet ist, einige Lichtstrahlen, die auf die anteriore Oberfläche einfallen, auf mindestens eine Stelle zu richten, die im Verhältnis zu dem Bild versetzt ist, um eine Wahrnehmung von visuellen Artefakten in einem peripheren Sehfeld zu verhindern,
**dadurch gekennzeichnet, dass** die Stelle eine Netzhautstelle ist.

**2.** IOL nach Anspruch 1, wobei eine primäre Fokussierungskraft, die durch eine Kombination der anterioren Oberfläche (14, 13, 46, 58, 67) und des mittleren Bereichs der posterioren Oberfläche (16, 15, 48, 58, 69) bereitgestellt wird, größer ist als eine jeweilige sekundäre Fokussierungskraft, die durch eine Kombination der anterioren Oberfläche und des peripheren Bereichs der posterioren Oberfläche bereitgestellt wird.

**3.** IOL nach Anspruch 1, wobei die anteriore Oberfläche einen Radius (14, 13, 46, 58, 67) im Verhältnis zu der optischen Achse in einem Bereich von ungefähr 2 mm bis ungefähr 4,5 mm aufweist.

**4.** IOL nach Anspruch 3, wobei der mittlere Bereich der posterioren Oberfläche (16, 15, 48, 58, 69) einen Radius im Verhältnis zu der optischen Achse in einem Bereich von ungefähr 1,5 mm bis ungefähr 4 mm aufweist.

**5.** IOL nach Anspruch 4, wobei der Randbereich (22, 19, 48b, 60, 71) eine Breite in einem Bereich von ungefähr 0,5 mm bis ungefähr 1 mm aufweist.

**6.** IOL nach Anspruch 4, wobei mindestens entweder die anteriore Oberfläche oder der mittlere Bereich der posterioren Oberfläche eine Abweichung von der Kugelgestalt aufweist, die durch eine konische Konstante in einem Bereich von ungefähr -10 bis ungefähr -100 **gekennzeichnet** ist.

**7.** IOL nach Anspruch 1, ferner umfassend eine Randfläche (18, 61), die sich zwischen den Grenzen der anterioren und posterioren Oberflächen erstreckt.

**8.** IOL nach Anspruch 1, wobei die Randfläche (18, 61) strukturiert ist, um darauf einfallendes Licht zu streuen.

**9.** IOL nach Anspruch 8, wobei die strukturierte Randfläche (18, 61) eine Vielzahl von Oberflächenwellungen (26) umfasst, die physikalische Oberflächenamplituden in einem Bereich von ungefähr 0,5 Mikrometer bis ungefähr 2 Mikrometern aufweist.

**10.** IOL nach Anspruch 1, ferner umfassend eine Fresnel-Linse (21), die auf dem peripheren Bereich der posterioren Oberfläche angeordnet ist.

**11.** IOL nach Anspruch 1, ferner umfassend eine beugende Struktur (60), die auf dem peripheren Bereich der posterioren Oberfläche angeordnet ist.

12. Intraokulare Linse (IOL) nach Anspruch 1 oder 11,
wobei die anteriore Oberfläche (14, 13, 46, 58, 67) und der mittlere Bereich zusammenwirkend mehrere Fokussierungskräfte bereitstellen, und der periphere Bereich (22, 19, 48b, 60, 71) der posterioren Oberfläche (16, 15, 48, 58, 69) dazu geeignet ist, mindestens einige darauf einfallende Lichtstrahlen auf eine Netzhautstelle zwischen einem Bild, das von der anterioren Oberfläche und dem mittleren Bereich gebildet wird, und einem zweiten peripheren Bild, das durch Lichtstrahlen gebildet wird, die in die IOL eindringen und an der IOL vorbeigehen, zu richten, um die Wahrnehmung von peripheren visuellen Artefakten zu verhindern.

13. Intraokulare Linse (IOL) nach Anspruch 1, wobei
der periphere Bereich (22, 19, 48b, 60, 71) eine ringförmige periphere Oberfläche umfasst, welche die posteriore Oberfläche (16, 15, 48, 58, 69) mindestens teilweise umgibt, und die anteriore Oberfläche und die posteriore Oberfläche zusammenwirkend eine Hauptfokussierungskraft bereitstellen, um ein Bild eines Sehfeldes auf der Netzhaut eines Auges eines Patienten, in dem die IOL implantiert ist, zu erzeugen, und wobei die ringförmige periphere Oberfläche dazu geeignet ist, um in Kombination mit der anterioren Oberfläche einige Lichtstrahlen, die auf der anterioren Oberfläche einfallen, mit einer sekundären Fokussierungskraft, die geringer ist als die Hauptbrechkraft, auf die Netzhaut zu richten, um die Wahrnehmung von visuellen Artefakten in einem peripheren Sehfeld zu verhindern.

14. IOL nach Anspruch 1 oder 13, wobei der periphere Bereich dazu geeignet ist, mindestens einige der Lichtstrahlen zu empfangen, die auf der anterioren Oberfläche in Winkeln in einem Bereich von ungefähr 50 bis ungefähr 80 Grad im Verhältnis zu der optische Achse einfallend sind.

15. IOL nach Anspruch 13, wobei die anteriore Oberfläche und die posteriore Oberfläche im Wesentlichen konvexe Formen aufweisen.

16. IOL nach Anspruch 15, wobei die ringförmige periphere Oberfläche eine im Wesentlichen konkave Form aufweist.

17. IOL nach Anspruch 2 oder 13, wobei die sekundäre Fokussierungskraft sich von der primären Fokussierungskraft um einen Faktor in einem Bereich von ungefähr 25 % bis ungefähr 75 % unterscheidet.

18. IOL nach Anspruch 13, wobei die sekundäre Fokussierungskraft eine beugende Fokussierungskraft umfasst.

19. IOL nach Anspruch 13, wobei die ringförmige, periphere Oberfläche und die posteriore Oberfläche eine angrenzende optische Oberfläche bilden.

20. Intraokulare Linse (IOL) nach Anspruch 1,
wobei der periphere Bereich (22, 19, 48b, 60, 71, 71') eine ringförmige Fokussierungsfläche umfasst, welche die posteriore Oberfläche umgibt, und wobei die ringförmige Fokussierungsfläche dazu geeignet ist, die Wahrnehmung von peripheren visuellen Artefakten zu verhindern, nachdem die IOL in ein Auge eines Patienten implantiert wurde.

21. IOL nach Anspruch 20, wobei die ringförmige Fokussierungsfläche Licht, das darauf einfällt auf eine oder mehrere Netzhautstellen richtet, die im Verhältnis zu einem Bild eines Sehfeldes, das zusammenwirkend durch die anterioren und posterioren Oberflächen gebildet wird, versetzt sind.

22. IOL nach Anspruch 20, wobei die ringförmige Fokussierungsfläche eine brechende Fokussierungskraft oder eine beugende Fokussierungskraft bereitstellt.

23. IOL nach Anspruch 20, wobei die ringförmige Fokussierungsfläche eine beugende Struktur (60), um die beugende Fokussierungskraft bereitzustellen, oder eine Fresnel-Linse (21) umfasst.

24. Intraokulare Linse (IOL) (63) nach Anspruch 1,
wobei der periphere Bereich (71, 71') ein oder mehrere Fokussierungselemente umfasst, welche die posteriore Oberfläche mindestens teilweise umgeben, um Licht in die Netzhaut zu richten, um die Wahrnehmung von visuellen Artefakten in einem peripheren Sehfeld zu verhindern.

25. IOL nach Anspruch 24, wobei die Fokussierungselemente Elementarlinsen (73) oder optische Rillen (73') umfassen.

**Revendications**

1. Lentille intraoculaire (IOL) (10, 11, 42, 54, 63), comprenant :

   une surface optique antérieure (14, 13, 46, 58, 67) et une surface optique postérieure (16, 15, 48, 58, 69) disposées autour d'un axe optique, ladite surface postérieure comportant une région centrale s'étendant vers une région périphérique (22, 19, 48b, 60, 71, 71'),
   dans laquelle la surface antérieure (14, 13, 46, 58, 67) et ladite région centrale sont adaptées pour former, en coopération, une image d'un champ de vision sur la rétine et ladite région périphérique (22, 19, 48b, 60, 71, 71') est adaptée pour diriger certains rayons de lumière frappant la surface antérieure vers au moins un emplacement décalé par rapport à ladite image de manière à empêcher la perception d'artefacts visuels dans un champ visuel périphérique, **caractérisée en ce que** ledit emplacement est un emplacement rétinien.

2. IOL selon la revendication 1, dans laquelle une puissance de focalisation principale fournie par une combinaison de ladite surface antérieure (14, 13, 46, 58, 67) et de ladite région centrale de la surface postérieure (16, 15, 48, 58, 69) est supérieure à une puissance de focalisation secondaire respective fournie par une combinaison de ladite surface antérieure et de ladite région périphérique de la surface postérieure.

3. IOL selon la revendication 1, dans laquelle ladite surface antérieure présente un rayon (14, 13, 46, 58, 67) par rapport audit axe optique dans une plage d'environ 2 mm à environ 4,5 mm.

4. IOL selon la revendication 3, dans laquelle ladite région centrale de la surface postérieure (16, 15, 48, 58, 69) présente un rayon par rapport audit axe optique dans une plage d'environ 1,5 mm à environ 4 mm.

5. IOL selon la revendication 4, dans laquelle ladite région périphérique (22, 19, 48b, 60, 71) a une largeur dans une plage d'environ 0,5 mm à environ 1 mm.

6. IOL selon la revendication 4, dans laquelle au moins l'une de ladite surface antérieure ou de ladite région centrale de la surface postérieure présente une asphéricité **caractérisée par** une constante conique dans une plage d'environ -10 à environ -100.

7. IOL selon la revendication 1, comprenant en outre une surface de bord (18, 61) s'étendant entre les frontières desdites surfaces antérieure et postérieure.

8. IOL selon la revendication 1, dans laquelle ladite surface de bord (18, 61) est texturée de manière à diffuser la lumière qui la frappe.

9. IOL selon la revendication 8, dans laquelle ladite surface de bord (18, 61) texturée comprend une pluralité d'ondulations de surface (26) ayant des amplitudes de surface physique dans une plage d'environ 0,5 micron à environ 2 microns.

10. IOL selon la revendication 1, comprenant en outre une lentille de Fresnel (21) disposée sur ladite région périphérique de la surface postérieure.

11. IOL selon la revendication 1, comprenant en outre une structure de diffraction (60) disposée sur ladite région périphérique de la surface postérieure.

12. Lentille intraoculaire (IOL) selon les revendications 1 ou 11,
    dans laquelle ladite surface antérieure (14, 13, 46, 58, 67) et ladite région centrale fournissent, en coopération, de multiples puissances de focalisation et ladite région périphérique (22, 19, 48b, 60, 71) de la surface postérieure (16, 15, 48, 58, 69) est adaptée pour diriger au moins certains rayons de lumière qui la frappent vers un emplacement rétinien entre une image formée par la surface antérieure et la région centrale et une deuxième image périphérique formée par les rayons de lumière entrant dans l'IOL qui ratent l'IOL de manière à empêcher la perception d'artefacts visuels périphériques.

13. Lentille intraoculaire (IOL) selon la revendication 1, dans laquelle
    la région périphérique (22, 19, 48b, 60, 71) comprend une surface périphérique annulaire entourant au moins partiellement ladite surface postérieure (16, 15, 48, 58, 69), et ladite surface antérieure et ladite surface postérieure fournissant, en coopération, une puissance de focalisation principale pour générer une image d'un champ de vision sur la rétine de l'oeil d'un patient dans lequel l'IOL est implantée, et dans laquelle ladite surface périphérique annulaire est adaptée pour diriger, en combinaison avec ladite surface antérieure, certains rayons de lumière frappant la surface antérieure vers la rétine avec une puissance de focalisation secondaire inférieure à ladite puissance principale de manière à empêcher la perception d'artefacts visuels dans un champ visuel périphérique.

14. IOL selon la revendication 1 ou 13, dans laquelle ladite région périphérique est adaptée pour recevoir au moins certains des rayons de lumière qui frappent la surface antérieure selon des angles dans une plage d'environ 50 à environ 80 degrés par rapport à l'axe optique.

15. IOL selon la revendication 13, dans laquelle ladite surface antérieure et ladite surface postérieure ont des formes sensiblement convexes.

16. IOL selon la revendication 15, dans laquelle ladite surface périphérique annulaire a une forme sensiblement concave.

17. IOL selon la revendication 2 ou 13, dans laquelle ladite puissance de focalisation secondaire diffère de ladite puissance de focalisation principale d'un facteur dans une plage d'environ 25 % à environ 75 %.

18. IOL selon la revendication 13, dans laquelle ladite puissance de focalisation secondaire comprend une puissance de focalisation par diffraction.

19. IOL selon la revendication 13, dans laquelle ladite surface périphérique annulaire et ladite surface postérieure forment une surface optique contiguë.

20. Lentille intraoculaire (IOL) selon la revendication 1,
dans laquelle la région périphérique (22, 19, 48b, 60, 71, 71') comprend une surface de focalisation annulaire entourant ladite surface postérieure, et dans laquelle ladite surface de focalisation annulaire est adaptée pour empêcher la perception d'artefacts visuels périphériques une fois que l'IOL est implantée dans l'oeil d'un patient.

21. IOL selon la revendication 20, dans laquelle ladite surface de focalisation annulaire dirige la lumière qui la frappe vers un ou plusieurs emplacements rétiniens décalés par rapport à une image d'un champ de vision formé en coopération par lesdites surfaces antérieure et postérieure.

22. IOL selon la revendication 20, dans laquelle ladite surface de focalisation annulaire fournit une puissance de focalisation par réfraction ou une puissance de focalisation par diffraction.

23. IOL selon la revendication 20, dans laquelle ladite surface de focalisation annulaire comprend une structure de diffraction (60) pour fournir ladite puissance de focalisation par diffraction, ou une lentille de Fresnel (21).

24. Lentille intraoculaire (IOL) (63) selon la revendication 1, dans laquelle
la région périphérique (71, 71') comprend un ou plusieurs éléments de focalisation entourant au moins partiellement la surface postérieure pour diriger la lumière vers la rétine de manière à empêcher la perception d'artefacts visuels dans un champ visuel périphérique.

25. IOL selon la revendication 24, dans laquelle lesdits éléments de focalisation comprennent de petites lentilles (73) ou des rainures optiques (73').

FIG. 1A

FIG. 1B

## FIG. 2

## FIG. 3

## FIG. 4

## FIG. 5

## FIG. 6A

## FIG. 6B

## FIG. 7

NORMALIZED IRRADIANCE PROFILES

SPHERICAL EDGE, R=100mm (CENTERED ON LENS AXIS)
TORIC ARC EDGE, R=100mm
6mm OD IOL MODEL, NO TAPER

## FIG. 8

FIG. 9

FIG. 10A

FIG. 10B

FIG. 10C

## FIG. 11A

## FIG. 11B

71'

69'

73'

**FIG. 11C**

**FIG. 12**

44

12

52

48b

50

48a

OA

48

48b

48

46

EP 2 152 202 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2005060031 A
- US 7156854 B
- US 00072804 A
- US 20060116763 A
- WO 11000770 A
- WO 2006060480 A
- US 5699142 A